# EUROPÄISCHE PATENTANMELDUNG

(11) **EP 0 733 346 A1**
(43) Veröffentlichungstag der Anmeldung: **25.09.1996**
(21) Anmeldenummer: 96103514.4
(22) Anmeldetag: 07.03.1996
(51) Int. Cl.: A61B 17/92, A61B 17/064, A61C 8/00, A61B 17/68

(54) **Heftgerät und Heftnagel für die Chirurgie**

(30) Priorität: 18.03.1995 DE 19509966
(71) Anmelder: Schütz, Frank Ullrich, 97082 Würzburg (DE)
(72) Erfinder: Schütz, Frank Ullrich, 97082 Würzburg (DE)
(74) Vertreter: Pöhner, Wilfried Anton, Dr.

(57) **Zusammenfassung**

Vorgeschlagen wird ein Heftgerät für die Chirurgie, vorzugsweise die Zahnchirurgie, zur Befestigung von für Gewebezellen impermeablen Membranen mit Heftnägeln an Knochen, wobei das Heftgerät einen Stößel (3) mit einer endseitigen Halterung (4) aufweist, in die ein Heftnagel mit seinem Kopf einschiebbar ist, der Stößel (3) unmittelbar oder über ein mechanisches Übertragungselement durch eine Federkraft axial in Richtung der Nagelhalterung (4) beweglich ist und die Entspannung der Feder (2) durch eine Auslösevorrichtung entriegelbar blockiert ist.

## Beschreibung

Die Erfindung bezieht sich auf ein Heftgerät für die Chirurgie, vorzugsweise die Zahnchirurgie, zur Befestigung von für Gewebezellen impermeablen Membranen mit Heftnägeln an Knochen sowie einen Heftnagel.

Zur Regeneration fehlender Knochensubstanz sind Verfahren nach dem Prinzip der "Guided Tissue Regeneration" bekannt, die insbesondere im Bereich der Implantologie angewandt werden, da das Vorliegen eines ausreichenden Volumens gesunder Knochensubstanz eine wesentliche Voraussetzung für den festen und dauerhaften Halt eines Implantats darstellt. Speziell bei der Implantation von Zahnprothesen im Kiefer tritt häufig die Situation ein, daß das Implantat unzureichend von Knochenmaterial umgeben ist und ein Knochenaufbau zu erfolgen hat. Dabei werden in den Bereich an einer Knochenoberfläche, in dem neue Knochensubstanz entstehen soll, an anderer Stelle entnommene Knochenpartikel verbracht oder der unterliegende Knochen angebohrt, um den Knochenaufbau bewirkende Osteozyten freizusetzen, und anschließend eine für Gewebezellen impermeable Membran aufgebracht (Schweiz Monatsschrift Zahn-med., Vol. 102, 1992, 1491 bis 1501). Da die Membran das Eindringen von Weichteilzellen, die den Knochenaufbau unterbinden, verhindert, ist unter ihr ein ungestörtes Wachstum des Knochens möglich, der jedoch nur in vorhandene Hohlräume hinein stattfindet, so daß eine ausreichende mechanische Festigkeit der Abdeckung notwendig ist. Im Stande der Technik erfolgt ihre Befestigung durch einhängen an der Prothese sowie durch Schrauben unmittelbar am Knochen. Dazu ist zunächst ein Loch in den Knochen zu bohren, die Membran zu perforieren, anschließend mit einer Schraube die Perforation zu durchstechen und die Schraube in das vorgebohrte Loch einzuführen. Anschließend erfolgt das Festschrauben im Knochenmaterial. Der gravierendste Nachteil dieser Vorgehensweise besteht darin, daß die Durchführung der einzelnen Arbeitsschritte einen erheblichen Zeitaufwand erfordert, der die körperliche Belastung des Patienten erhöht. Weiterhin ergibt sich häufig das Problem, daß die Membran mit der eingedrehten Schraube verdreht wird und den Bereich, in dem der Knochen aufzubauen ist, nur noch unvollständig abdeckt, was den Erfolg der Operation gefährdet. Schließlich sind für einen hinreichenden Kraftschluß mit dem Schraubenzieher Schrauben mit hinreichend großen Köpfen zu verwenden, die die Zahl möglicher Befestigungsorte einschränken; beispielsweise lassen sich im Stande der Technik Membranbefestigungen nicht im Bereich des Kiefernkammes anbringen, da ide Schraubenköpfe hier die Schleimhaut mit der Zeit perforieren würden.

Vor diesem Hintergrund hat sich die Erfindung zur Aufgabe gestellt, ein Gerät zu entwickeln, mit dem sich eine Membran in wesentlich kürzerer Zeit am Knochen befestigen läßt, wobei die Zahl möglicher Befestigungsorte erhöht und die Gefahr einer Verschiebung der Abdeckung während der Befestigung vermindert wird.

Diese Aufgabe wird erfindungsgemäß dadurch gelöst, daß das Heftgerät einen Stößel mit einer endseitigen Halterung aufweist, in die ein Heftnagel mit seinem Kopf einschiebbar ist, der Stößel unmittelbar oder über ein mechanisches Übertragungselement durch eine Federkraft axial in Richtung der Nagelhalterung beweglich ist und die Entspannung der Feder durch eine Auslösevorrichtung entriegelbar blockiert ist.

Die Erfindung sieht vor, daß die Fixierung der Membran am Knochen durch Heftnägel anstelle der bisher verwendeten Schrauben erfolgt. Zu diesem Zweck ist der Heftnagel mit seinem Kopf in eine Halterung eingeschoben, die endseitig an einem Stößel des Heftgerätes angeordnet ist. Der Stößel ist durch eine Federkraft axial in Richtung der Nagelhalterung beweglich. Dabei kann die Kraft der Feder, für die sowohl Druck- als auch Zugfedern einsetzbar sind, unmittelbar oder über ein mechanisches Übertragungselement, z.B. einen Hebel, auf den Stößel übertragen werden. Die Entspannung der Feder ist entriegelbar durch eine Auslösevorrichtung blockiert, die am Stößel, dem Übertragungselement oder der Feder selbst angreifen kann. Eine Membran wird mit dem Heftgerät befestigt, indem das Heftgerät mit dem eingeschobenen Nagel an den vorgesehenen Befestigungsort verbracht, in Eintreibrichtung ausgerichtet und die Auslösevorrichtung betätigt wird. Durch Entspannung der Feder wird der Stößel in Eintreibrichtung beschleunigt, bis der Nagel auf die Membran trifft und diese mit seiner Spitze durchdringt, d.h. eine vorherige Perforation der Membran ist unnötig. Durch die Bewegungsenergie des Stößels wird der Nagel bis in den Knochen eingetrieben und fixiert die Membran auf diese Weise. Je nach Ausführung des Heftgerätes löst sich der Nagel bereits im Verlauf des Eintreibvorganges oder wird beim Abziehen des Heftgerätes aus der Halterung gezogen.

Mit dem vorgeschlagenen Heftgerät ist eine wesentlich schnellere Fixierung der Membran am Knochen erreichbar. Viele bisher notwendige Verfahrensschritte, im speziellen das Vorbohren eines Loches sowie die Perforation der Membran entfallen. Damit werden die Belastungen des Patienten im Verlauf einer Operation wesentlich verringert und die Operationskosten gesenkt. Darüber hinaus zeigt der Heftnagel ein günstigeres Fixierungsverhalten als eine Schraube, d.h. ein Verdrehen der Membran ist bei einem Nagel ausgeschlossen, da er ohne Rotation in den Knochen eingetrieben wird. Weil der Kopf eines Nagels im Gegensatz zu einer Schraube nicht zur Übertragung eines Drehmomentes genutzt wird, läßt er sich wesentlich kleiner und flacher ausführen, so daß die möglichen Fixierungspunkte der Membran räumlich wesentlich erweitert werden und beispielsweise den Kiefernkamm einschließen.

Bei einer bevorzugten Ausgestaltung der Erfindung erfolgt die Kraftübertragung von der Feder auf den Stößel über einen Bolzen. Dieser ist endseitig an der Feder befestigt, kolinear der Stößelachse beabstandet zum Stößel angeordnet und in dessen Richtung verschieblich. Nach Betätigung der Auslösevorrichtung wird der Bolzen in Richtung des Stößels beschleunigt, trifft vergleichbar einem Hammer auf diesen auf und treibt den Nagel so in den Knochen ein. Neben einer einfachen und platzsparenden Bauform besteht bei dieser Ausgestaltung der Erfindung insbesondere der Vorteil, daß das Heftgerät mit der Nagelspitze unmittelbar am Eintreibpunkt auf die Membran aufsetzbar und damit eine sehr genaue Positionierung des Nagels gewährleistet ist.

Bei Kraftübertragung mit einem Bolzen ist eine Haltefeder zweckmäßig, an der der Stößel befestigt ist. Sie gewährleistet, daß der Ort des Stößels vor dem Auftreffen des Bolzens festgelegt ist und demzufolge eine gute Kontrolle über den Eintreibvorgang besteht. Gleichzeitig ist der Stößel hinreichend beweglich, so daß ein Eintreiben des Nagels möglich bleibt.

Weiterhin wird vorgeschlagen, daß die Stößelbewegung endseitig durch einen Anschlag begrenzt ist. Ein nagelseitiger Anschlag hat dabei einerseits zum Ziel, die Eintreibtiefe des Nagels definiert zu begrenzen, und andererseits die nach ihrem Erreichen verbleibende Bewegungsenergie des Stößels aufzunehmen, damit diese nicht auf den Knochen übertragen wird. Speziell im Zusammenhang mit einer Haltefeder des Stößels ist auch ein bolzenseitiger Anschlag des Stößels sinnvoll, an dem er gegebenenfalls unter Einfluß der Kraftwirkung der Haltefeder anliegt, und der die Ausgangslage des Stößels bestimmt. Die Position des Anschlages ist vorzugsweise verstellbar und gestattet so die Einstellung unterschiedlicher Eintreibtiefen des Nagels.

In einer Weiterbildung der Erfindung ist darüber hinaus die Ausgangsposition des Bolzens oder der Befestigungsort der Antriebsfeder verstellbar. Wie auch beim Anschlag kann die Verstellung beispielsweise mit Hilfe einer Rändelmutter erfolgen. Die Verstellbarkeit bietet die Möglichkeit, die Bewegungsenergie des Stößels zu variieren, um Anpassungen an individuelle oder lokal unterschiedliche Knochenstrukturen vorzunehmen oder einen noch teilweise herausstehenden Nagel mit einem zweiten Schlag geringerer Stärke vollständig in den Knochen einzutreiben.

In einer geeigneten Ausführung eines verstellbaren Bolzens weist dieser ein Arretierungsraster auf. Die zugehörige Auslösevorrichtung umfaßt einen senkrecht der Bolzenachse beweglichen Riegel, an dem der Bolzen unter dem Einfluß der Kraft der Antriebsfeder mit einer Gegenfläche anliegt. Entlang seiner Achse ist der Bolzen mit mehreren gleichartigen Gegenflächen versehen, so daß mehrere Rastmöglichkeiten mit jeweils unterschiedlicher potentieller Energie des Bolzens bestehen.

Ein am Bolzen befestigter Griff, der aus dem Hefter heraussteht, dient zum Spannen der Feder. Er kann seitlich oder endseitig am Bolzen angebracht sein, wobei die letztere Bauform die Handhabung des Hefters vereinfacht, wenn in schlecht zugänglichen Bereichen, etwa im hinteren Teil des Mundraumes, gearbeitet wird.

Ein bevorzugter, mit dem Heftgerät zu befestigender Heftnagel weist einen senkrecht der Eintreibrichtung flächig ausgebildeten Kopf auf, um zu vermeiden, daß die befestigte Membran über den Kopf hinweg vom Nagel abrutscht. Dabei gestattet eine geringe Dicke des Kopfes, den Nagel auch in räumlich beschränkten Positionen, etwa im Bereich des Kiefernkammes, einzusetzen. Der Schaft läuft endseitig spitz zu, so daß sowohl das Durchdringen der Membran als auch das Eindringen in den Knochen erleichtert wird. Aufgrund der Elastizität der Knochensubstanz gewährleistet ein mit Widerhaken versehener Schaft einen sicheren und dauerhaften Halt des Heftnagels.

Nach Abschluß des Knochenaufbaus sind Membran und Befestigungselemente wieder zu entfernen. Zu diesem Zweck ist vorgesehen, den Kopf des Nagels im äußeren Bereich seiner Unterseite mit dem Ziel abzuschrägen, ein Untergreifen mit einer Zange oder einem anderen Werkzeug zu erleichtern. Insbesondere bei Nägeln, die sehr fest im Knochen sitzen, wird auf diese Weise die Entfernung vereinfacht.

Für das Material des Nagels sind elementare oder legierte, gewebeverträgliche Metalle geeignet, um Abstoßungsreaktionen auszuschließen. Insbesondere Titan und seine Legierungen haben sich im Bereich der Chirurgie seit langem bewährt. Alternativ sind vom Körper resorbierbare Materialien einsetzbar, so daß die Notwendigkeit beseitigt wird, den Nagel nach erfolgtem Knochenaufbau entfernen zu müssen.

Der Halt des Nagels erfolgt nahezu ausschließlich in der äußeren, festen Knochenschicht, der Corticalis, während die poröse Struktur des Knocheninneren durch den Nagel geschädigt wird, ohne seine Befestigung zu fördern. Da die Corticalisdicke des menschlichen Kiefernknochens etwa 2 bis 5 mm beträgt, wird vorgeschlagen, daß die Schaftlänge im Bereich von 2 bis 3 mm liegt.

Weitere Einzelheiten, Merkmale und Vorteile der Erfindung lassen sich dem nachfolgenden Beschreibungsteil entnehmen, in dem anhand der Zeichnung ein Ausführungsbeispiel der Erfindung näher erläutert wird.

Die Zeichnung zeigt in prinzipienhafter Darstellung
- Figur 1: ein erfindungsgemäßes Heftgerät
- Figur 2: einen geeigneten Heftnagel

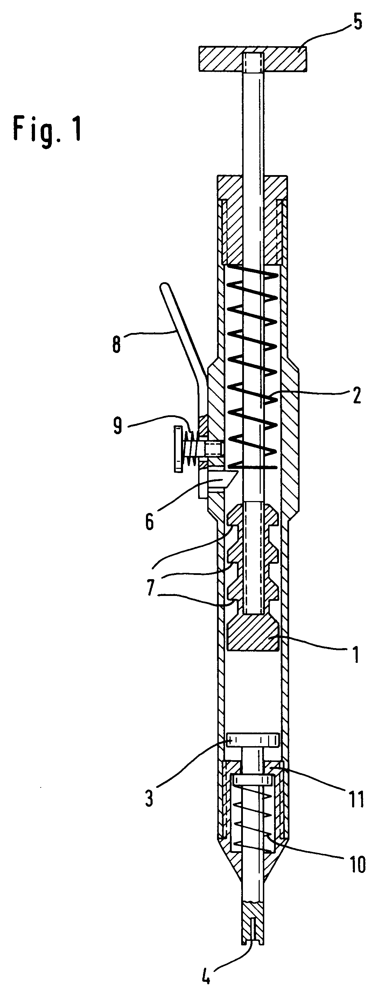

Das in Figur 1 dargestellte Heftgerät besteht in seinem grundsätzlichen Aufbau aus einem Bolzen (1), der durch die Kraft einer Feder (2) gegen einen Stößel (3) beweglich ist, an dessen Ende sich eine Halterung (4) befindet, die zur Aufnahme des Kopfes eines Heftnagels vorgesehen ist. Die Feder (2) wird gespannt, indem der mit dem Bolzen (1) verbundene Griff (5) herausgezogen wird, bis der Riegel (6) an einer Gegenfläche (7) des Bolzens (1) einrastet und ihn damit festhält. Mehrere entlang der Bewegungsachse des Bolzens (1) angeordnete Gegenflächen (7) bilden ein Arretierungsraster, das es ermöglicht, die Feder (2) unterschiedlich stark zu spannen. Die Auslösevorrichtung des Heftgerätes wird von einem Hebel (8) gebildet, an dem der Riegel (6) befestigt ist und unter dem Einfluß einer Rückstellfeder (9) an eine der Gegenflächen (7) gedrückt wird.

Bei Betätigung des Hebels (8) wird der Bolzen (1) entriegelt und durch die Kraft der gespannten Feder (2) in Richtung des Stößels (3) bewegt, auf den er nach Art eines Hammers auftrifft, so daß sich der Stößel (3) seinerseits schlagartig in Richtung der Halterung (4) verschiebt. Ein in der Halterung (4) befestigter Nagel läßt sich durch diese Stößelbewegung in einen Knochen eintreiben und kann dabei zur Befestigung einer Membran dienen. Der Stößel (3) wird durch eine Haltefeder (10) in Richtung auf den Bolzen (1) zu gehalten, so daß für seine Bewegung jeweils der maximal mögliche Weg zur Verfügung steht. Die Begrenzung der Weglänge erfolgt dabei durch einen Anschlag (11), der verhindert, daß nach Erreichen der vorgesehenen Eintreibtiefe noch vorhandene, überschüssige Bewegungsenergie des Stößels auf den Knochen übertragen wird und Beschädigungen verursacht.

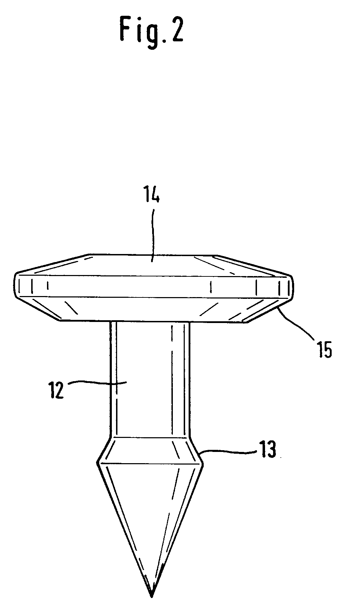

Figur 2 zeigt einen zweckmäßigen, im Zusammenhang mit dem Heftgerät verwendbaren Heftnagel. Sein Schaft (12), dessen Länge vorzugsweise etwa der Dicke der Corticalisschicht entspricht, läuft endseitig spitz zu, um ein Eindringen in den Knochen zu erleichtern. Widerhaken (13) im Bereich des Schaftes gewähren einen sicheren und dauerhaften Halt im Knochen. Der Kopf (14) des Heftnagels ist flächig ausgeführt und gestattet somit die sichere Befestigung einer Membran am Knochen, ohne daß diese vom Schaft (12) über den Kopf (14) hinweg vom Nagel abgleiten kann. Im äußeren Bereich seiner Unterseite weist der Nagel eine Schräge (15) auf, die sein Untergreifen mit einem Werkzeug erleichtert, um ihn zusammen mit der Membran nach einer erfolgreichen Knochenregeneration wieder zu entfernen.

## Patentansprüche

1. Heftgerät für die Chirurgie, vorzugsweise die Zahnchirurgie, zur Befestigung von für Gewebezellen impermeablen Membranen mit Heftnägeln an Knochen, **dadurch gekennzeichnet**, daß
- das Heftgerät einen Stößel (3) mit einer endseitigen Halterung (4) aufweist, in die ein Heftnagel mit seinem Kopf einschiebbar ist,
- der Stößel (3) unmittelbar oder über ein mechanisches Übertragungselement durch eine Federkraft axial in Richtung der Nagelhalterung (4) beweglich ist,
- und die Entspannung der Feder (2) durch eine Auslösevorrichtung entriegelbar blockiert ist.

2. Heftgerät nach Anspruch 1, **dadurch gekennzeichnet**, daß die Feder (2) und ein endseitig an ihr befestigter Bolzen (1) kolinear der Stößelachse beabstandet zum Stößel (3) angeordnet sind und der Bolzen (1) in Richtung des Stößels (3) verschieblich ist.

3. Heftgerät nach Anspruch 2, **dadurch gekennzeichnet**, daß der Stößel (3) an einer Haltefeder (10) befestigt ist.

4. Heftgerät nach einem der vorhergehenden Ansprüche, **dadurch gekennzeichnet**, daß die Stößelbewegung endseitig durch einen Anschlag (11) begrenzt ist.

5. Heftgerät nach Anspruch 4, **dadurch gekennzeichnet**, daß die Position des Anschlages (11) verstellbar ist.

6. Heftgerät nach einem der Ansprüche 2 bis 5, **dadurch gekennzeichnet**, daß die Position, in der der Bolzen (1) verriegelt ist, oder der Befestigung der Feder (2) verstellbar ist.

7. Heftgerät nach Anspruch 6, **dadurch gekennzeichnet**, daß die Auslösevorrichtung einen senkrecht der Bolzenachse beweglichen Riegel (6) umfaßt, an dem der Bolzen (1) mit einer von mehreren gleichartigen Gegenflächen (7) entlang seiner Achse anliegt.

8. Heftgerät nach einem der Ansprüche 2 bis 7, **dadurch gekennzeichnet**, daß der Bolzen (1) mit einem Griff (5) versehen ist, der aus dem Hefter heraus ragt.

9. Heftgerät nach einem der vorhergehenden Ansprüche mit einem in einen Knochen eintreibbaren Heftnagel, **dadurch gekennzeichnet**, daß der Nagel einen senkrecht der Eintreibrichtung flächig ausgebildeten Kopf (14) und einen endseitig spitz zulaufenden Schaft (12) aufweist.

10. Heftgerät nach Anspruch 9, **dadurch gekennzeichnet**, daß der Schaft (12) mit Widerhaken (13) versehen ist.

11. Heftgerät nach einem der Ansprüche 9 oder 10, **dadurch gekennzeichnet**, daß der Kopf (14) im äußeren Bereich seiner Unterseite abgeschrägt ist.

12. Heftgerät nach einem der Ansprüche 9 bis 11, **dadurch gekennzeichnet**, daß der Nagel aus einem gewebeverträglichen biokompatiblen Metall besteht.

13. Heftgerät nach einem der Ansprüche 9 bis 11, **dadurch gekennzeichnet**, daß das Nagelmaterial resorbierbar ist.

14. Heftnagel nach einem der Ansprüche 9 bis 13, **dadurch gekennzeichnet**, daß die Schaftlänge etwa 2 bis 3 mm beträgt.
